# EUROPEAN PATENT SPECIFICATION

(11) **EP 3 772 285 B1**
(45) Date of publication and mention of the grant of the patent: **28.06.2023**
(21) Application number: 19190810.2
(22) Date of filing: 08.08.2019
(51) Int. Cl.: A23D 9/05, A23L 2/39, B01D 1/02, B01D 1/18, B01J 2/04, A61K 9/14, A61K 9/16

(54) **PULVERIZATION OF A SUBSTANCE**
PULVERISIERUNG EINER SUBSTANZ
PULVÉRISATION D'UNE SUBSTANCE

(43) Date of publication of application: 10.02.2021
(73) Proprietor: Air Liquide Deutschland GmbH, 40235 Düsseldorf (DE); L'Air Liquide, Société Anonyme pour l'Etude et l'Exploitation des Procédés Georges Claude, 75007 Paris (FR)
(72) Inventor: Angenheister de Freitas, Alexander, 41460 Neuss (DE); Böckling, Matthias, 47807 Krefeld (DE); Kasten, Christine, 31363 Jüchen (DE); Kinghorst, Fabian, 47228 Duisburg (DE)
(74) Representative: Keenway Patentanwälte Neumann Heine Taruttis

(56) References cited:
- EP-A2- 0 214 441
- EP-A2- 0 393 963
- EP-B1- 2 367 434
- WO-A1-2012/130468
- US-A- 3 892 880
- US-A1- 2019 126 163
- US-A1- 2019 183 068

## Description

The present invention is directed to a nozzle installation and a method for spraying a substance that is supposed to be pulverized. Further, the present invention is directed to a use of the nozzle installation. Also, the present invention is directed to a device and a method for pulverizing a substance.

From the prior art various devices and methods for pulverizing substances are known. In particular, it is known to pulverize fat by spraying the fat with a nozzle into a spray tower. Therein, the sprayed fat is subjected to a cryogenic such that the fat droplets generated by the nozzle are frozen to form a powder.

According to prior art solutions for such a pulverization of substances, the nozzle often becomes clogged. This is particularly true with substances having a comparatively high melting point and/or a small solidification range. The substance can solidify within the nozzle such that a solid block of the substance is formed that blocks the nozzle. This might cause significant downtime. Even if the nozzle is not entirely clogged such that the process has to be interrupted, solidification of the substance is disadvantageous. This is due to the fact that even a partial clogging of the nozzle can result in fluctuations in pressure. These, in turn, can lead to fluctuations in the size of the obtained powder particles. That is, clogging of the nozzle can result in a nonuniform particle size distribution or even in an interruption of the process. Fluctuation in pressure can occur in particular at the beginning or end of a process.

From US 2019/0183068 A1 a fogging apparatus for atomizing a medium is known. From EP 0 393 963 A2 an apparatus for producing solid particles of fat is known. EP 2 367 434 B1 discloses a system ("set-up B") and related methods for preparing an edible fat powder, the system comprising: an autoclave 16 equipped with a stirrer 17 and with a top connection 18 to CO2 for pressuring the system; a nozzle 12 connected to the autoclave 16 via a tube 20 and spraying into an expansion vessel 11. The expansion vessel 11 comprises an inlet 13 for additional CO2 cooling. The autoclave 16 is heated with an oil bath with heat transfer fluid and the tube 20 and the nozzle 12 are kept at the desired temperature by a heating tape using a Thyristor to adjust its temperature. In an example, the temperature of the tube 20 and the nozzle 12 is set above the melting temperature of the fat mixture. In operation, the expansion vessel 11 is cooled to the desired temperature with CO2 gas over inlet 13 and a CO2-fat melt mixture is fed from the autoclave 16 and sprayed through the nozzle 12 at atmospheric pressure in the expansion vessel 11 with external CO2 gas (13) used for additional cooling to keep the expansion vessel at the desired temperature. The sprayed CO2-melt mixture is rapidly solidified into a powder collected in a barrel (15) below the expansion vessel.

It is, therefore, an object of the present invention to overcome at least in part the disadvantages known from prior art and, in particular, to provide a nozzle installation and a method for spraying a substance that is supposed to be pulverized, a use of the nozzle installation as well as a device and a method for pulverizing a substance by means of which the substance can be pulverized in a more reliable manner.

These objects are solved by the features of the independent claims. Dependent claims are directed to preferred embodiments of the present invention.

According to the invention a nozzle installation for spraying a substance that is supposed to be pulverized is provided. The nozzle installation comprises:
- a first inlet for the substance,
- a second inlet for a driving medium,
- an outlet, through which the substance can be driven by the driving medium,
- a heating line that is arranged such that the substance can be heated between the first inlet and the outlet by the heating line,
- a first temperature sensor arranged at the outlet, and
- a control unit configured for controlling the heating power of the heating line based on a first temperature measured by the first temperature sensor.

By means of the described nozzle installation the substance can be sprayed in order to pulverize the substance in a subsequent step. In particular, fat can be the substance. For example, the fat can be a fractionated palm fat, rapseed oil or sunflower oil.

The described nozzle installation is preferably intended to be used in the production of food products or pharmaceuticals. The pulverized substance can be the final product that can be sold, for example an emulsifier or a fat product. Alternatively, the pulverized substance can be an intermediate product such as an ingredient for a food product or a pharmaceutical. For example, the pulverized substance can be a baking ingredient, a pharmaceutically active substance or a food supplement or a feed additive.

The product is preferably supplied to the nozzle installation, in particular to the first inlet thereof, in its liquid state. Preferably, the product is supplied at a temperature that is 5 to 15°C above the melting point of the substance. Preferably, the substance is supplied to the nozzle installation at a temperature between 70 and 150°C. For example, the product can be supplied from a substance supply such as a tank by means of a heated piping.

The nozzle installation preferably comprises a housing. The housing could also be referred to as a nozzle. In addition to the housing, the nozzle installation comprises the control unit. The nozzle installation comprises two inlets and an outlet. The inlets and the outlet are preferably openings within the housing. The housing of the nozzle installation is preferably manufactured by means of additive manufacturing.

The substance can be sprayed by ejecting the substance out of the outlet. Therefore, the substance is provided to the nozzle installation via the first inlet. The substance can be driven by a driving medium. Therefore, it is preferred that the driving medium is supplied to the nozzle installation at a sufficiently high pressure. In particular, this pressure is preferably higher than the atmospheric pressure. The driving medium can be supplied to the nozzle installation via the second inlet. The driving medium can be supplied to the nozzle installation from a driving medium supply via a respective piping. The driving medium is preferably a gaseous medium. For example, the driving medium can be nitrogen and/or carbon dioxide.

Within the nozzle installation, in particular within the housing thereof, the substance and the driving medium can come into contact with each other. If the flow of the driving medium is sufficiently high, the flow of the driving medium can generate a respective flow of the substance. The substance can be entrained by the driving medium. The nozzle installation can be considered a two-fluid nozzle.

By means of the described nozzle installation the substance can be pulverized in a particularly reliable manner. In particular, clogging of the nozzle installation can be prevented particularly well. This is achieved by heating the substance. Therefore, the heating line is provided. The heating line is preferably arranged within the housing of the nozzle installation. The heating line is preferably arranged such that the substance can be heated by means of the heating line along the way of the substance from the first inlet to the outlet. Preferably, the heating line extends from the first inlet to the outlet such that the substance can be heated along the entire way from the first inlet to the outlet. By means of the heating line, the substance can be heated. That means that at least the substance can be heated. The driving medium can also be heated by the heating line. However, the advantageous effect of the heating mainly occurs due to the heating of the substance. Therefore, it is not necessary that the driving medium is also heated. Nevertheless, if the substance and the driving medium are mixed or at least in close proximity to each other, heating the substance may necessarily be accompanied by heating of the driving medium.

The substance can be brought into contact with the driving medium directly downstream of the inlets. In that case, heating of the substance and of the driving medium can occur jointly along the entire way from the inlets to the outlet. Alternatively, the substance and the driving medium can be guided separately from each other through a part of the housing. In that case, the substance can be heated upstream of the point where the substance and the driving medium are brought into contact with each other. This does not exclude that simultaneously the driving medium is heated and/or that the substance and the driving medium are heated downstream of the point where the substance and the driving medium are brought into contact with each other.

Due to the heating, solidification of the substance can be prevented, in particular at the outlet. Thus, clogging of the nozzle can be prevented. Thereby, interruptions of the process can be avoided, and a particularly uniform particle size distribution can be obtained. The described nozzle installation can be configured for the production of particle sizes in the range of 60 to 1000 µm. A production of 1,000 kg/h may be achieved.

These positive effects achievable due to the avoiding of a clogging of the nozzle can even be enhanced due to the temperature control performed by the control unit. This control is based on a temperature measured by a first temperature sensor. This temperature is referred to as the first temperature. The first temperature sensor is arranged at the outlet. This might be considered the point of use because the temperature of the substance at the outlet is the temperature at which the substance is sprayed. Depending on the first temperature the heating power of the heating line is controlled by the control unit.

Due to the exact temperature of the substance prior to spraying a particularly uniform particle size distribution can be achieved. This is due to the fact that the temperature of the substance is closely related to its viscosity. Hence, different temperatures of the substance are accompanied by respectively different surface tensions of droplets of the substance that leave the outlet. The surface tension, in turn, is closely related to the cooling speed, at which the droplets can be frozen by subjection to a cryogenic medium. Thus, the temperature of the substance at the outlet is closely related to the particle size. A uniform temperature distribution of the droplets leaving the outlet accordingly leads to a uniform distribution of sizes of the obtained particles.

According to a preferred embodiment of the nozzle installation the heating line is a heating wire.

The heating wire is preferably made with a material that heats up when an electric currents flows therethrough. The heating wire could also be referred to as a heating resistance. The heating by means of a heating wire occurs due to Joule heat.

Using a heating wire is advantageous because the heating power thereof directly depends on the applied electric current. Changing an electric current can be done easily and quickly. Thus, using a heating wire makes a simple and fast temperature control possible.

According to a further preferred embodiment of the nozzle installation the heating line is a heating conduit for a heating medium.

The heating conduit can be flown through by the heating medium. Thus, the temperature of the heating medium can be transferred to the heating conduit and, eventually, to the environment of the heating conduit. Preferably, the heating medium is a thermal oil. The heating medium can be provided to the heating conduit via a heating conduit inlet and can be extracted from the heating conduit via a heating conduit outlet. Connected to the heating conduit inlet and to the heating conduit outlet can be a heating medium supply. The heating medium supply is preferably configured for circulating the heating medium through the heating conduit.

Control of the heating power of the heating conduit can be performed by controlling the flow rate of the heating medium through the heating conduit and/or by controlling the temperature at which the heating medium is supplied to the heating conduit inlet.

According to a further preferred embodiment of the nozzle installation the heating line is configured as a spiral.

The spiral is preferably arranged such that an axis of the spiral is parallel to an axis of the housing of the nozzle installation. This is particularly true if the housing of the nozzle installation is rotationally symmetric.

Due to a spiral-shaped heating line, the substance can be heated within the nozzle installation in a particularly efficient manner.

According to a further preferred embodiment the nozzle installation further comprises a pressure sensor arranged at the outlet, wherein the control unit is configured for controlling the heating power of the heating line further based on a pressure measured by the pressure sensor.

It has been found that also the pressure surrounding the outlet of the nozzle installation can have an influence on the particle size of the obtained particles. This refers to the pressure of the atmosphere outside the outlet. In particular, this can be the pressure within a spray tower. Accordingly, it is preferred that the pressure sensor is arranged such that the pressure outside the outlet of the nozzle installation can be measured.

Control of the heating power based on the first temperature and on the pressure can be performed, for example, in that a setpoint for the first temperature is selected based on the pressure. Therefore, a predefined table with respective values for the first temperature corresponding to various pressure values can be obtained from experiments.

According to a further preferred embodiment the nozzle installation further comprises a second temperature sensor that is arranged at the heating line, wherein the control unit is configured for controlling the heating power of the heating line further based on a second temperature measured by the second temperature sensor.

The temperature measured by the second temperature sensor is referred to as the second temperature, in order to distinguish the temperatures measured by the first and second temperature sensors. That is, there is no first temperature measured by the second temperature sensor.

The fact that the second temperature sensor is arranged at the heating line is supposed to be understood such that the second temperature can be considered a measure for the temperature of the heating line. Hence, the second temperature sensor can be arranged directly on a surface of the heating line or even within the heating line.

The substance might have a maximum temperature that is not supposed to be exceeded. By means of the second temperature sensor, such an overheating can be prevented. This is particularly true if the heating line has a nonuniform temperature distribution as is the case with a heating conduit flown through by a heating medium. A heating conduit usually has its highest temperature at the heating conduit inlet and its lowest temperature at the heating conduit outlet. Thus, it is preferred that the second temperature sensor is arranged at the heating conduit inlet.

The control unit is preferably configured such that the temperature is controlled generally based on the first temperature with the boundary condition that the second temperature does not exceed a predefined threshold. Thus, unless the second temperature exceeds the threshold the temperature control is based on the first temperature. However, if the threshold is exceeded, the heating power is reduced.

According to a further preferred embodiment the nozzle installation further comprises a static mixer between the first inlet and the second inlet on the one hand and the outlet on the other hand.

The static mixer is preferably configured for mixing the driving medium and the substance. Thereby, the substance can be accelerated particularly well by the driving medium. Preferably, the static mixer comprises elements for deviating the flow of the substance and the driving medium such that they are mixed particularly well. A thorough mixing of the substance and the driving medium can result in a particularly uniform temperature distribution in the substance and the driving medium.

The static mixer is preferably arranged at the outlet. Upstream of the mixer, the substance and the driving medium are preferably guided through the housing of the nozzle in a separate manner. That is, the substance is brought into contact with the driving medium only after the substance has been heated. This does not exclude that the substance and the driving medium are further heated after being brought into contact with each other.

The substance and the driving medium are preferably brought into contact with each other in the static mixer and not upstream thereof. However, the substance and the driving medium can also be brought into contact with each other upstream of the static mixer.

According to a further aspect of the invention a use of the described nozzle installation for spraying a fat as the substance that is supposed to be pulverized is provided.

The details and advantages disclosed for the claimed nozzle installation also apply to the claimed use.

According to a further aspect of the invention a device for pulverizing a substance is provided. The device comprises:
- a nozzle installation according to one of the preceding claims,
- a spray tower, into which the substance can be sprayed by the nozzle installation, and
- a cryogen injector arranged within the spray tower for subjecting the substance sprayed into the spray tower by the nozzle installation to a cryogenic medium.

The details and advantages disclosed for the claimed nozzle installation and its use also apply to the claimed device.

The spray tower preferably comprises a chamber, into which the substance can be sprayed via the outlet of the nozzle installation. The nozzle installation is preferably arranged on top of the spray tower such that the substance can be sprayed into the spray tower in a downward direction. Within the chamber the sprayed substance can be subjected to the cryogenic medium. The cryogenic medium is preferably liquid nitrogen or carbon dioxide. By subjecting the sprayed substance to the cryogenic medium the particles of the substance can freeze and thereby form a powder. This can be the case, in particular due to turbulences within the spray tower.

The substance can be subjected to the cryogenic medium by spraying the cryogenic medium into the chamber by means of the cryogen injector. Therefore, it is preferred that the cryogen injector is arranged at the outlet of the nozzle installation or at least in close proximity thereto. That is, the cryogen injector is preferably arranged at the top of the spray tower. The cryogen injector is preferably a spray ring.

The powder can be extracted at the bottom of the spray tower, in particular via a rotary feeder. Downstream thereof, a packaging installation may be provided for packaging the obtained product. Alternatively, installations for further processes can be provided. The powder is preferably extracted from the spray tower at a temperature in the range of 5 to 10°C.

The device preferably further comprises a substance supply for supplying the substance to the first inlet of the nozzle installation by a respective piping, preferably comprising a pump. The piping is preferably heated and/or comprises a heater for heating the substance prior to introducing the substance into the nozzle installation.

The device preferably further comprises a driving medium supply for supplying the driving medium to the second inlet of the nozzle installation via a respective piping. Also, the device preferably further comprises a cryogen supply for supplying the cryogenic medium to the cryogen injector via a respective piping.

The device preferably further comprises an exhaust gas purifier that is connected to the top of the spray tower. The exhaust gas purifier preferably comprises a cyclone, by means of which gas exhausted from the spray tower can be cleaned before being exhausted to the environment. The cyclone is preferably connected to the top of the spray tower via a respective piping. From the cyclone the purified air can be exhausted to the environment, while waste material can be collected in its solid state.

According to a further aspect of the invention a method for spraying a substance that is supposed to be pulverized is provided. The method comprises:
a) introducing the substance into a nozzle installation,
b) introducing a driving medium into the nozzle installation,
c) heating the substance by means of a heating line arranged within the nozzle installation,
d) driving the substance through an outlet of the nozzle installation by the driving medium,
e) measuring a first temperature at the outlet, and
f) controlling a heating power at which the substance is heated in step c) based on the first temperature measured in step e).

The details and advantages disclosed for the claimed nozzle installation, its use and the device also apply to the claimed method for spraying a substance that is supposed to be pulverized.

Steps a) and b) are preferably performed simultaneously. Steps c) and d) are preferably performed in sequence after steps a) and b). This refers to the order of steps that a certain amount of the substance and of the driving medium is subjected to. That is, the substance and the driving medium are first introduced into the nozzle installation, the substance is then heated and eventually let out of the outlet. However, the flow of the substance and of the driving medium through the nozzle installation is preferably continuous. That is, while a certain amount of the substance is let out of the outlet, a different amount of the substance is introduced into the nozzle installation and a further different amount of the substance is heated. Insofar, steps a) to d) may also be considered to be performed simultaneously.

Steps e) and f) describe the control of the heating power. The temperature control is supposed to be performed simultaneously parallel to steps a) to d). However, if only a certain amount of the substance is considered, the temperature thereof is measured at the outlet. That is, step e) is performed after or during step d) and step f) is performed after step e). Nevertheless, measuring the temperature and using the measured values as an input for the control is a continuous process, such that steps e) and f) may also be considered to be performed simultaneously to each other and to the steps a) to d).

According to a preferred embodiment of the method the temperature control in step f) is performed depending on a desired particle size of the substance at the outlet.

The obtained particle size is related to the temperature of the substance at the outlet of the nozzle installation. Hence, a set point for this temperature can be chosen according to the desired particle size.

According to a further preferred embodiment of the method inert conditions are maintained within the nozzle installation.

Preferably, inert conditions are maintained during the entire process. In particular, inert conditions are maintained within the housing of the nozzle. Therefore, it is preferred that the housing of the nozzle is confined tightly. Further, the driving medium is preferably an inert gas such as nitrogen.

Inert conditions make the treatment of food products or of ingredients for food products possible.

According to a further preferred embodiment of the method the substance is a fat.

According to a further preferred embodiment of the method the substance has a melting point above 70°C.

Fats with a melting point above 70°C can be considered high-melting fats. In particular for these substances the described advantages can be achieved.

According to a further aspect of the invention a method for pulverizing a substance is provided. The method comprises:
A) spraying the substance into a spray tower by the described method for spraying a substance that is supposed to be pulverized, and
B) subjecting the substance sprayed into the spray tower according to step A) to a cryogenic medium injected into the spray tower.

The details and advantages disclosed for the claimed nozzle installation, its use, the device and the method for spraying a substance that is supposed to be pulverized also apply to the claimed method for pulverizing a substance.

Steps A) and B) are preferably performed after each other. This refers to the order of steps that a certain amount of the substance is subjected to. Since the process is preferably performed in a continuous manner, steps A) and B) may also be considered to be performed simultaneously. While a certain amount of the substance is subjected to the cryogenic medium according to step B), a different amount of the substance can be sprayed into the spray tower according to step A).

The specification, in particular taken together with the figures, explains the invention further and specifies particularly preferred embodiments of the invention. Particularly preferred variants of the invention and the technical field will now be explained in more detail with reference to the enclosed figures. It should be noted that the exemplary embodiment shown in the figures is not intended to restrict the invention. The figures are schematic and may not be to scale. The figures display:
- Fig. 1:: a schematic sectional side view of a nozzle installation according to a preferred embodiment of the present invention,
- Fig. 2:: a schematic side view of a device for pulverizing a substance according to a preferred embodiment of the present invention that comprises the nozzle installation of Fig. 1,
- Fig. 3:: a schematic flow diagram of a method for spraying a substance that is supposed to be pulverized according to the present invention, wherein the nozzle installation of Fig. 1 can be used, and
- Fig. 4:: a schematic flow diagram of a method for pulverizing a substance, wherein the device of Fig. 2 can be used.

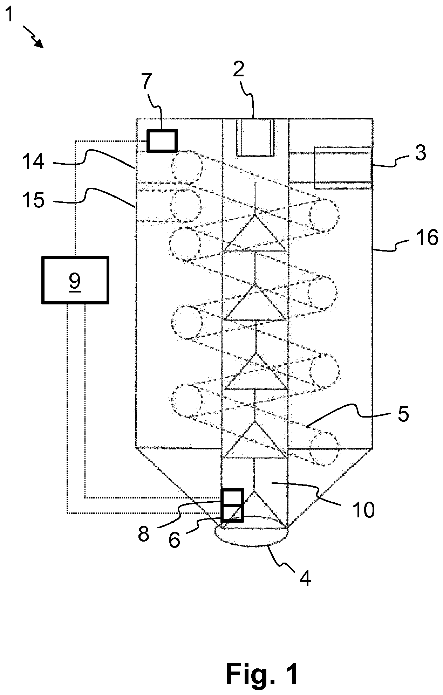

Fig. 1 shows a nozzle installation 1 for spraying a substance that is supposed to be pulverized. The nozzle installation 1 comprises a first inlet 2 for the substance and a second inlet 3 for a driving medium. Further, the nozzle installation 1 comprises an outlet 4, through which the substance can be driven by the driving medium.

The inlets 2,3 and the outlet 4 are arranged at a housing 16 of the nozzle installation 1.

A heating line 5 that is configured in a spiral manner is arranged such that the substance can be heated between the first inlet 2 and the outlet 4 by the heating line 5. In the shown embodiment the heating line 5 is a heating conduit for a heating medium. Accordingly, the heating line 5 has a heating conduit inlet 14 and a heating conduit outlet 15. Alternatively, the heating line 5 could also be a heating wire (not shown in the figures).

At the outlet 4, a first temperature sensor 6 and a pressure sensor 8 are arranged. At the heating line 5, in particular at the heating conduit inlet 14, a second temperature sensor 7 is provided.

The nozzle installation 1 further comprises a control unit 9 that is configured for controlling the heating power of the heating line 5 based on a first temperature measured by the first temperature sensor 6, a second temperature measured by the second temperature sensor 7 and a pressure measured by the pressure sensor 8. Therefore, the control unit 9 is connected to the sensors 7,8,9, which is indicated by dotted lines.

The nozzle installation 1 further comprises a static mixer 10 between the first inlet 2 and the second inlet 3 on the one hand and the outlet 4 on the other hand. In the shown embodiment the static mixer 10 is arranged at the outlet 4.

The nozzle installation 1 can be used for spraying a fat as the substance that is supposed to be pulverized.

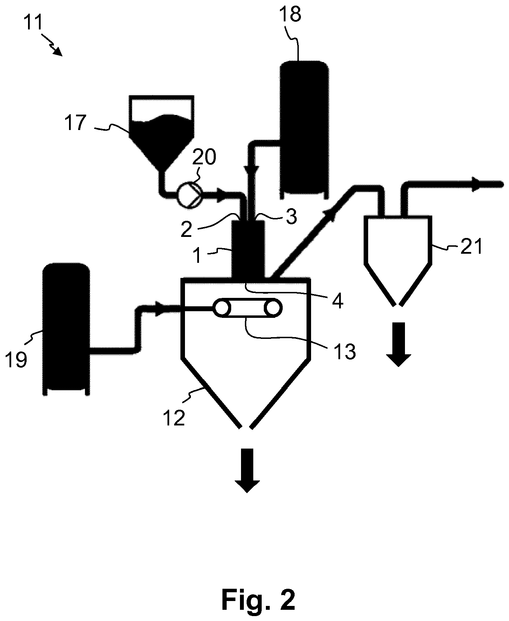

Fig. 2 shows a device 11 for pulverizing a substance. The device 11 comprises a nozzle installation 1 that is configured as shown in Fig. 1. The inlets 2,3 and the outlet 4 are indicated in Fig. 2. The device 11 further comprises a spray tower 12, into which the substance can be sprayed by the nozzle installation 1. Therefore, the nozzle installation 1 is connected to the spray tower 12 via the outlet 4. The substance is provided to the first inlet 2 of the nozzle installation 1 from a substance supply 17 via a respective piping comprising a pump 20. The driving medium can be provided to the second inlet 3 of the nozzle installation 1 from a driving medium supply 18 via a respective piping.

Further, the device 11 comprises a cryogen injector 13 arranged within the spray tower 12 for subjecting the substance sprayed into the spray tower 12 by the nozzle installation 1 to a cryogenic medium. The cryogenic medium can be provided to the spray tower 12 from a cryogen supply 19 via a respective piping.

The pulverized substance can be extracted at the bottom of the spray tower 12 as indicated by an arrow.

The device 11 further comprises an exhaust gas purifier 21 that is connected to the top of the spray tower 12 via a respective piping. Collected waste material can be extracted from the bottom of the exhaust gas purifier 21 as indicated by an arrow.

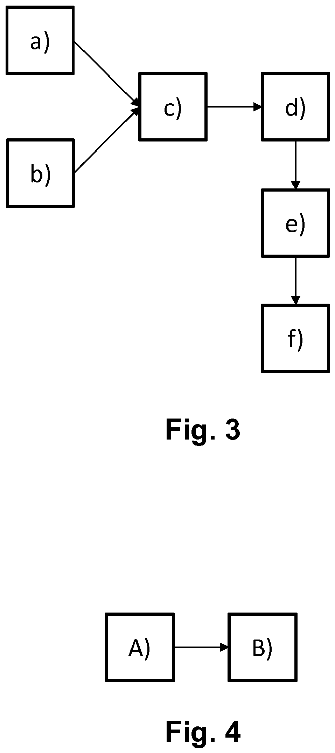

Fig. 3 shows a schematic flow diagram of a method for spraying a substance that is supposed to be pulverized. The substance can be a fat and/or have a melting point above 70°C. The nozzle installation 1 of Fig. 1 can be used for the method.

The method comprises:
a) introducing the substance into a nozzle installation 1,
b) introducing a driving medium into the nozzle installation 1,
c) heating the substance by means of a heating line 5 arranged within the nozzle installation 1,
d) driving the substance through an outlet 4 of the nozzle installation 1 by the driving medium,
e) measuring a first temperature at the outlet 4, and
f) controlling a heating power at which the substance is heated in step c) based on the first temperature measured in step e) and depending on a desired particle size of the substance at the outlet 4.

During the performance of the method, inert conditions are maintained within the nozzle installation 1.

As can be seen from Fig. 3, steps a) and b) are preferably performed simultaneously. Steps c) and d) are performed in sequence after steps a) and b). Therein, it should be noted that the illustration of Fig. 3 refers to the order of steps that a certain amount of the substance and of the driving medium is subjected to.

Since here only a certain amount of the substance is considered, step e) is depicted below step d) in Fig. 3. After the temperature is measured, the measured value can be used as an input for the control according to step f). Thus, step f) is depicted downstream of step e).

Fig. 4 shows a schematic flow diagram of a method for pulverizing a substance, wherein the device 11 of Fig. 2 can be used. The method comprises:
A) spraying the substance into a spray tower 12 by the method according to Fig. 3, wherein the nozzle installation 1 of Fig. 1 can be used, and
B) subjecting the substance sprayed into the spray tower 12 according to step A) to a cryogenic medium injected into the spray tower 12 of the device 11 of Fig. 2.

As can be seen from Fig. 4, steps A) and B) are performed after each other. This refers to the order of steps that a certain amount of the substance is subjected to.

By means of the described methods, the described nozzle installation 1 and the described device 11 pulverization of a substance such as a fat can be performed in a particularly reliable manner. Due to a controlled heating of the substance prior to spraying the substance into a spray tower 12, a particular uniform particle size distribution can be achieved and clogging of a nozzle installation 1 can be prevented.

### List of reference numerals

- 1: nozzle installation
- 2: first inlet
- 3: second inlet
- 4: outlet
- 5: heating line
- 6: first temperature sensor
- 7: second temperature sensor
- 8: pressure sensor
- 9: control unit
- 10: static mixer
- 11: device
- 12: spray tower
- 13: cryogen injector
- 14: heating conduit inlet
- 15: heating conduit outlet
- 16: housing
- 17: substance supply
- 18: driving medium supply
- 19: cryogen supply
- 20: pump
- 21: exhaust gas purifier

## Claims

1. Nozzle installation (1) for spraying a substance that is supposed to be pulverized, comprising:
- a first inlet (2) for the substance,
- a second inlet (3) for a driving medium,
- an outlet (4), through which the substance can be driven by the driving medium,
- a heating line (5) that is arranged such that the substance can be heated between the first inlet (2) and the outlet (4) by the heating line (5),
- a first temperature sensor (6) arranged at the outlet (4), and
- a control unit (9) configured for controlling the heating power of the heating line (5) based on a first temperature measured by the first temperature sensor (6).

2. Nozzle installation (1) according to claim 1, wherein the heating line (5) is a heating wire.

3. Nozzle installation (1) according to claim 1, wherein the heating line (5) is a heating conduit for a heating medium.

4. Nozzle installation (1) according to one of the preceding claims, wherein the heating line (5) is configured as a spiral.

5. Nozzle installation (1) according to one of the preceding claims, further comprising a pressure sensor (8) arranged at the outlet (4), wherein the control unit (9) is configured for controlling the heating power of the heating line (5) further based on a pressure measured by the pressure sensor (8).

6. Nozzle installation (1) according to one of the preceding claims, further comprising a second temperature sensor (7) that is arranged at the heating line (5), wherein the control unit (9) is configured for controlling the heating power of the heating line (5) further based on a second temperature measured by the second temperature sensor (7).

7. Nozzle installation (1) according to one of the preceding claims, further comprising a static mixer (10) between the first inlet (2) and the second inlet (3) on the one hand and the outlet (4) on the other hand.

8. Use of a nozzle installation (1) according to one of the preceding claims for spraying a fat as the substance that is supposed to be pulverized.

9. Device (11) for pulverizing a substance, comprising:
- a nozzle installation (1) according to one of the preceding claims,
- a spray tower (12), into which the substance can be sprayed by the nozzle installation (1), and
- a cryogen injector (13) arranged within the spray tower (12) for subjecting the substance sprayed into the spray tower (12) by the nozzle installation (1) to a cryogenic medium.

10. Method for spraying a substance that is supposed to be pulverized, comprising:
a) introducing the substance into a nozzle installation (1),
b) introducing a driving medium into the nozzle installation (1),
c) heating the substance by means of a heating line (5) arranged within the nozzle installation (1),
d) driving the substance through an outlet (4) of the nozzle installation (1) by the driving medium,
e) measuring a first temperature at the outlet (4), and
f) controlling a heating power at which the substance is heated in step c) based on the first temperature measured in step e).

11. Method according to claim 10, wherein the temperature control in step f) is performed depending on a desired particle size of the substance at the outlet (4).

12. Method according to claim 10 or 11, wherein inert conditions are maintained within the nozzle installation (1).

13. Method according to one of claims 10 to 12, wherein the substance is a fat.

14. Method according to one of claims 10 to 13, wherein the substance has a melting point above 70°C.

15. Method for pulverizing a substance, comprising:
A) spraying the substance into a spray tower (12) by a method according to one of the claims 10 to 14, and
B) subjecting the substance sprayed into the spray tower (12) according to step A) to a cryogenic medium injected into the spray tower (12).

## Patentansprüche

1. Düseneinrichtung (1) zum Versprühen eines Stoffs, der pulverisiert werden soll, umfassend:
- einen ersten Einlass (2) für den Stoff,
- einen zweiten Einlass (3) für ein Treibmedium,
- einen Auslass (4), durch den der Stoff von dem Treibmedium getrieben werden kann,
- eine Heizleitung (5), die so angeordnet ist, dass der Stoff zwischen dem ersten Einlass (2) und dem Auslass (4) von der Heizleitung (5) erhitzt werden kann,
- einen ersten Temperatursensor (6), der an dem Auslass (4) angeordnet ist, und
- eine Steuereinheit (9), die zum Steuern der Heizleistung der Heizleitung (5) auf Grundlage einer von dem ersten Temperatursensor (6) gemessenen ersten Temperatur gestaltet ist.

2. Düseneinrichtung (1) gemäß Anspruch 1, wobei die Heizleitung (5) ein Heizdraht ist.

3. Düseneinrichtung (1) gemäß Anspruch 1, wobei die Heizleitung (5) ein Heizkanal für ein Heizmedium ist.

4. Düseneinrichtung (1) gemäß einem der vorstehenden Ansprüche, wobei die Heizleitung (5) als Spirale gestaltet ist.

5. Düseneinrichtung (1) gemäß einem der vorstehenden Ansprüche, ferner umfassend einen Drucksensor (8), der an dem Auslass (4) angeordnet ist, wobei die Steuereinheit (9) zum Steuern der Heizleistung der Heizleitung (5) ferner auf Grundlage eines von dem Drucksensor (8) gemessenen Drucks gestaltet ist.

6. Düseneinrichtung (1) gemäß einem der vorstehenden Ansprüche, ferner umfassend einen zweiten Temperatursensor (7), der an der Heizleitung (5) angeordnet ist, wobei die Steuereinheit (9) zum Steuern der Heizleistung der Heizleitung (5) ferner auf Grundlage einer von dem zweiten Temperatursensor (7) gemessenen zweiten Temperatur gestaltet ist.

7. Düseneinrichtung (1) gemäß einem der vorstehenden Ansprüche, ferner umfassend einen statischen Mischer (10) zwischen dem ersten Einlass (2) und dem zweiten Einlass (3) einerseits und dem Auslass (4) andererseits.

8. Verwendung einer Düseneinrichtung (1) gemäß einem der vorstehenden Ansprüche zum Versprühen eines Fetts als der Stoff, der pulverisiert werden soll.

9. Vorrichtung (11) zum Pulverisieren eines Stoffs, umfassend:
- eine Düseneinrichtung (1) gemäß einem der vorstehenden Ansprüche,
- einen Sprühturm (12), in den der Stoff von der Düseneinrichtung (1) gesprüht werden kann, und
- einen Kryogeninjektor (13), der in dem Sprühturm (12) angeordnet ist, zum Unterwerfen des von der Düseneinrichtung (1) in den Sprühturm (12) gesprühten Stoffs an ein kryogenes Medium.

10. Verfahren zum Versprühen eines Stoffs, der pulverisiert werden soll, umfassend:
a) Einführen des Stoffs in eine Düseneinrichtung (1),
b) Einführen eines Treibmediums in die Düseneinrichtung (1),
c) Erhitzen des Stoffs mithilfe einer Heizleitung (5), die in der Düseneinrichtung (1) angeordnet ist,
d) Treiben des Stoffs durch einen Auslass (4) der Düseneinrichtung (1) durch das Treibmedium,
e) Messen einer ersten Temperatur an dem Auslass (4) und
f) Steuern der Heizleistung, mit der der Stoff bei Schritt c) erhitzt wird, auf Grundlage der bei Schritt e) gemessenen ersten Temperatur.

11. Verfahren gemäß Anspruch 10, wobei die Temperatursteuerung bei Schritt f) abhängig von einer gewünschten Partikelgröße des Stoffs an dem Auslass (4) durchgeführt wird.

12. Verfahren gemäß Anspruch 10 oder 11, wobei inerte Bedingungen innerhalb der Düseneinrichtung (1) gehalten werden.

13. Verfahren gemäß einem der Ansprüche 10 bis 12, wobei der Stoff ein Fett ist.

14. Verfahren gemäß einem der Ansprüche 10 bis 13, wobei der Stoff einen Schmelzpunkt von über 70 °C aufweist.

15. Verfahren zum Pulverisieren eines Stoffs, umfassend:
A) Sprühen des Stoffs in einen Sprühturm (12) durch ein Verfahren gemäß einem der Ansprüche 10 bis 14 und
B) Unterwerfen des gemäß Schritt A) in den Sprühturm (12) gesprühten Stoffs an ein in den Sprühturm (12) eingespritztes kryogenes Medium.

## Revendications

1. Installation de buse (1) destinée à pulvériser une substance qui est censée être pulvérisée, comprenant :
- une première entrée (2) pour la substance,
- une deuxième entrée (3) pour un milieu d'entraînement,
- une sortie (4), à travers laquelle la substance peut être entraînée par le milieu d'entraînement,
- une ligne de chauffage (5) qui est disposée de telle sorte que la substance peut être chauffée entre la première entrée (2) et la sortie (4) par la ligne de chauffage (5),
- un premier capteur de température (6) disposé à la sortie (4), et
- une unité de régulation (9) configurée pour réguler la puissance de chauffage de la ligne de chauffage (5) sur la base d'une première température mesurée par le premier capteur de température (6).

2. Installation de buse (1) selon la revendication 1, dans laquelle la ligne de chauffage (5) est un fil chauffant.

3. Installation de buse (1) selon la revendication 1, dans laquelle la ligne de chauffage (5) est un conduit de chauffage pour un milieu chauffant.

4. Installation de buse (1) selon une des revendications précédentes, dans laquelle la ligne de chauffage (5) est configurée comme une spirale.

5. Installation de buse (1) selon une des revendications précédentes, comprenant en outre un capteur de pression (8) disposé à la sortie (4), dans laquelle l'unité de régulation (9) est configurée pour réguler la puissance de chauffage de la ligne de chauffage (5) également sur la base d'une pression mesurée par le capteur de pression (8).

6. Installation de buse (1) selon une des revendications précédentes, comprenant en outre un deuxième capteur de température (7) qui est disposé au niveau de la ligne de chauffage (5), dans laquelle l'unité de régulation (9) est configurée pour réguler la puissance de chauffage de la ligne de chauffage (5) également sur la base d'une deuxième température mesurée par le deuxième capteur de température (7).

7. Installation de buse (1) selon une des revendications précédentes, comprenant en outre un mélangeur statique (10) entre la première entrée (2) et la deuxième entrée (3) d'une part, et la sortie (4) d'autre part.

8. Utilisation d'une installation de buse (1) selon une des revendications précédentes pour la pulvérisation d'une graisse en tant que substance qui est censée être pulvérisée.

9. Dispositif (11) destiné à pulvériser une substance, comprenant :
- une installation de buse (1) selon une des revendications précédentes,
- une tour de pulvérisation (12), à l'intérieur de laquelle la substance peut être pulvérisée par l'installation de buse (1), et
- un injecteur de fluide cryogénique (13) disposé à l'intérieur de la tour de pulvérisation (12) pour soumettre la substance pulvérisée à l'intérieur de la tour de pulvérisation (12) par l'installation de buse (1) à un milieu cryogénique.

10. Procédé destiné à pulvériser une substance qui est censée être pulvérisée, comprenant :
a) l'introduction de la substance à l'intérieur d'une installation de buse (1),
b) l'introduction d'un milieu d'entraînement à l'intérieur de l'installation de buse (1),
c) le chauffage de la substance au moyen d'une ligne de chauffage (5) disposée à l'intérieur de l'installation de buse (1),
d) l'entraînement de la substance à travers une sortie (4) de l'installation de buse (1) par le milieu d'entraînement,
e) la mesure d'une première température à la sortie (4), et
f) la régulation d'une puissance de chauffage à laquelle la substance est chauffée à l'étape c) sur la base de la première température mesurée à l'étape e).

11. Procédé selon la revendication 10, dans lequel la régulation de température à l'étape f) est effectuée en fonction d'une taille de particules souhaitée de la substance à la sortie (4).

12. Procédé selon la revendication 10 ou 11, dans lequel des conditions inertes sont maintenues à l'intérieur de l'installation de buse (1).

13. Procédé selon une des revendications 10 à 12, dans lequel la substance est une graisse

14. Procédé selon une des revendications 10 à 13, dans lequel la substance a un point de fusion au-dessus de 70 °C.

15. Procédé destiné à pulvériser une substance, comprenant :
A) la pulvérisation de la substance à l'intérieur d'une tour de pulvérisation (12) par un procédé selon une des revendications 10 à 14, et
B) la soumission de la substance pulvérisée à l'intérieur de la tour de pulvérisation (12) selon l'étape A) à un milieu cryogénique injecté à l'intérieur de la tour de pulvérisation (12).
